(19) Europäisches Patentamt
European Patent Office
Office européen des brevets

(11) EP 3 659 575 A1

# (12) EUROPÄISCHE PATENTANMELDUNG

(43) Veröffentlichungstag:
**03.06.2020 Patentblatt 2020/23**

(51) Int Cl.:
***A61K 6/083*** *(2006.01)* ***C08F 2/38*** *(2006.01)*

(21) Anmeldenummer: **18208916.9**

(22) Anmeldetag: **28.11.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Ivoclar Vivadent AG
9494 Schaan (LI)**

(72) Erfinder:
- **GEBHARDT, Benjamin
9472 Grabs (CH)**
- **MOSZNER, Norbert
9495 Triesen (LI)**

(74) Vertreter: **Uexküll & Stolberg
Partnerschaft von
Patent- und Rechtsanwälten mbB
Beselerstraße 4
22607 Hamburg (DE)**

## (54) PHOTOPOLYMERISIERBARE DENTALE KOMPOSITE MIT SCHNELLER AUSHÄRTUNG UND GERINGER SCHRUMPFSPANNUNG

(57) Radikalisch polymerisierbare Werkstoff, der (a) 0,01 bis 5 Gew.-% mindestens eines Übertragungsreagenzes, (b) 5 bis 60 Gew.-% mindestens eines multifunktionellen (Meth)acrylates oder eine Mischung von mono- und multifunktionellen (Meth)-acrylaten, (c) 0,01 bis 3,0 Gew.-% einer Mischung mindestens eines monomolekularen und mindestens eines bimolekularen Photoinitiators, (d) 30 bis 90 Gew.-% mindestens eines Füllstoffs, und (e) gegebenenfalls Additiv(e) enthält, wobei der Werkstoff als Übertragungsreagenz (a) mindestens ein Allylsulfon der Formel I und/oder einen Vinylsulfonester der Formel II enthäl t.

EP 3 659 575 A1

## Beschreibung

Die vorliegende Erfindung betrifft lichthärtend radikalisch polymerisierbare Werkstoffe, die sich insbesondere als dentale Füllungskomposite, Zemente für Inlays, Onlays, Kronen oder Brücken und als Verblendmaterialien eignen.

Dentale Komposite, die z.B. als Kompositzement oder als direktes Füllungsmaterial zur Herstellung von Inlays, Onlays, Kronen oder als Verblendwerkstoff eingesetzt werden, enthalten eine polymerisierbare organische Matrix und einen oder mehreren Füllstoffe, die meist mit einem polymerisationsfähigen Haftvermittler oberflächenmodifiziert sind. Je nach Art der Füllstoffe, der Monomermatrix und der Anwendung kann der Füllstoffgehalt zwischen 30 bis 90 Gew.-% variieren, wobei Zemente im Vergleich zu Füllungskompositen einen geringeren Füllstoffgehalt aufweisen. Die polymerisierbare organische Matrix wird auch als Harz bezeichnet.

Die polymerisierbare organische Matrix enthält in der Regel eine Mischung von Monomeren, Initiator-Komponenten, Stabilisatoren und Pigmenten. Als Harze werden meistens Mischungen von Dimethacrylaten eingesetzt. Beispiele hierfür sind die hochviskosen Dimethacrylate 2,2-Bis[4-(2-hydroxy-3-methacryloyl-oxypropyl)phenyl]propan (Bis-GMA) und 1,6-Bis-[2-methacryloyl-oxyethoxycarbonylamino]-2,4,4-trimethylhexan (UDMA) oder die als Verdünnermonomere genutzten niedrigerviskosen Dimethacrylate Bismethacryloyloxymethyltricyclo[5.2.1.]decan (TCDMA), Decandiol-1,10-dimethacrylat ($D_3MA$) und Triethylenglycoldimethacrylat (TEGDMA).

Bei der radikalischen Polymerisation von dentalen Kompositen kommt es aufgrund des Polymerisationsschrumpfes ($\Delta V_P$) der eingesetzten Monomere zu einer Volumenkontraktion, was zur sehr nachteiligen Randspaltbildung bei Zahnfüllungen führen kann.

Während der Polymerisation von monofunktionellen Methacrylaten, wie z.B. Methylmethacrylat (MMA) ($\Delta V_P$ = 21,0 Vol.-%), führt die Polymerisationsschrumpfung nicht zum Aufbau einer Polymerisationsschrumpfungskraft (PSK), da die Verringerung des Volumens durch einfaches Fließen der gebildeten Makromoleküle kompensiert werden kann. Im Falle der vernetzenden Polymerisation von multifunktionellen Methacrylaten bildet sich aber bereits innerhalb weniger Sekunden am sogenannten Gelpunkt, d.h. schon bei geringem Monomerumsatz, ein dreidimensionales Polymernetzwerk aus, weshalb der Polymerisationsschrumpf nicht mehr durch viskoses Fließen kompensiert werden kann und das Material mit zunehmendem Monomerumsatz eine erhebliche PSK aufbaut. Diese führt zu Spannungen und unter Umständen auch zu Rissen im Material oder zur Abhebung vom Substrat. Dabei ist die Entwicklung der PSK oder entsprechender Spannungen bei Füllungskompositen von mehreren weiteren Faktoren abhängig, u.a. von der Höhe der Volumenkontraktion während der Polymerisation (Härtung bzw. Nachhärtung), den viskoelastischen Eigenschaften (Elastizitätsmodul und Modulaufbau), der Glasübergangstemperatur ($T_G$) des Polymers, der Viskosität und dem Fließverhalten, der Polymerisationskinetik, der Polymernetzwerkbildung (Harzfunktionalität, Vernetzungsdichte, Anteil an cyclischen Strukturen, Polymerisationsgeschwindigkeit, Temperatur, Monomer- und Doppelbindungsumsatz), der Art der Härtung und der Art der Restauration (Schichtdicke, Kavitätengeometrie) abhängig. Eine besonders hohe Schrumpfungsspannung wird bei der Lichthärtung beobachtet (vgl. R. R. Braga, R. Y. Ballester, J. L. Ferracane, Dent. Mater. 21 (2005) 962-970; J. W. Stansbury, Dent. Mater. 28 (2012) 13-22).

Es wurden zahlreiche Strategien zur Reduktion der PSK verfolgt. Das betrifft klinische Methoden, wie z.B. die Inkrement-Schicht-Technik, die Verwendung von Kavitätenlacken mit niedrigem E-Modul zur Bildung einer stressabsorbierenden Schicht oder das Vorerwärmen der Komposite zur Verbesserung der Fließeigenschaften. Der Einsatz von Monomeren mit ringöffnend polymerisierbaren Gruppen oder die Verwendung von Vernetzern mit photo- oder thermolabilen Spacern kann ebenfalls zu Kompositen mit geringer PSK führen.

Außerdem wurde versucht, die PSK durch den Zusatz von hyperverzweigten Monomeren, Nanogelen oder Nanotubes sowie von Low-Profile-Additiven oder expandierbaren Füllstoffen zu verringern.

Die US 2012/0295228 A1 offenbart radikalisch polymerisierbare Dentalmaterialien, die ethylenisch ungesättigte Monomere mit Disulfidgruppen enthalten, die als Additions-Fragmentierungs-Materialien wirksam sind und die die PSK reduzieren sollen.

Die WO 2015/057413 A1 offenbart Additions-Fragmentierungs-Oligomere die Disulfidgruppen in Allylpositionen enthalten. Diese können radikalisch polymerisierbaren Materialien zugesetzt werden, wobei die labilen Bindungen während der Polymerisation gespalten und neu geknüpft werden können. Damit soll die PSK reduziert werden.

Die WO 2015/041863 A1 offenbart vernetzende Additions-Fragmentierungs-Oligomere, die Trithiocarbonat-Strukturen enthalten. Die Oligomere können radikalisch polymerisierbaren Materialien zugesetzt werden. Sie bewirken eine Vernetzung der Materialien, wobei die Vernetzungen labil sind und während der Polymerisation gespalten und neu geknüpft werden können. Auf diese Weise soll die PSK verringert werden.

Nachteilig ist, dass die beschriebenen schrumpfungsspannungsarmen Komposite nach dem Stand der Technik relativ langsam aushärten und dementsprechend eine relativ lange Belichtungszeit zur Härtung benötigen.

Der Erfindung liegt die Aufgabe zugrunde, polymerisierbare Dentalwerkstoffe bereitzustellen, die zur Aushärtung eine geringe Belichtungszeit benötigen und die nach der Härtung für dentale Anwendungen geeignete mechanische Eigenschaften und insbesondere eine geringe Polymerisationsschrumpfungskraft (PSK) aufweisen.

Die Aufgabe wird erfindungsgemäß durch radikalisch polymerisierbare Werkstoffe gelöst, die

(a) 0,01 bis 5 Gew.-% mindestens eines Übertragungsreagenzes,
(b) 5 bis 60 Gew.-% mindestens eines multifunktionellen (Meth)-acrylates oder einer Mischung von mono- und multifunktionellen (Meth)acrylaten,
(c) 0,01 bis 3,0 Gew.-% einer Kombination mindestens eines monomolekularen und mindestens eines bimolekularen Photoinitiators,
(d) 30 bis 90 Gew.-% mindestens eines Füllstoffs, und
(e) gegebenenfalls Additiv(e) enthalten.

Der Werkstoff enthält als Übertragungsreagenz (a) mindestens ein Allylsulfon der Formel I

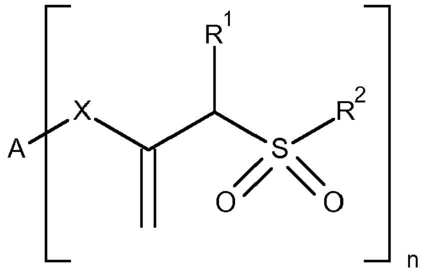

Formel I

,

in der die Variablen die folgenden Bedeutungen haben:

A H; -CN; ein Phenylrest, der einen oder mehrere Substituenten, wie $-CH_3$, $-C_2H_5$, -OH, $-OCH_3$, $-O-COCH_3$, eine polymerisationsfähige Vinyl-, (Meth)acryloyloxy- oder (Meth)-acrylamidgruppe tragen kann, oder ein aliphatischer linearer oder verzweigter $C_1$-$C_{20}$-Alkylen- oder $C_1$-$C_{20}$-Alkyl-Rest, der durch ein oder mehrere 1,4-Phenylengruppen, Urethangruppen, O oder S unterbrochen sein kann, der einen oder mehrere Substituenten, wie -OH oder $-OCH_3$, tragen kann und der endständig eine Tri-$C_1$-$C_4$-alkoxysilylgruppe oder eine polymerisationsfähige Vinyl-, (Meth)acryloyloxy- oder (Meth)acrylamidgruppe tragen kann;
$R^1$ jeweils unabhängig voneinander H, ein aliphatischer linearer oder verzweigter $C_1$-$C_9$-Alkyl-Rest, Tolyl oder Phenyl;
$R^2$ jeweils unabhängig voneinander ein Phenylrest, der einen oder mehrere Substituenten, wie $-CH_3$, $-C_2H_5$, -OH, $-OCH_3$, $-O-COCH_3$, eine polymerisationsfähige Vinyl-, (Meth)acryloyloxy-, (Meth)acrylamidgruppe, $-C(=CH_2)-COOR^3$ oder $-C(=CH_2)-CO-NR^4R^5$ tragen kann; oder ein aliphatischer linearer oder verzweigter $C_1$-$C_{20}$-Alkyl-Rest, der durch O oder S unterbrochen sein kann und der endständig eine polymerisationsfähige Vinyl-, (Meth)acryloyloxy-, (Meth)-acrylamidgruppe, $-C(=CH_2)-COOR^3$ oder $-C(=CH_2)-CO-NR^4R^5$ tragen kann, wobei $R^{3-5}$ unabhängig voneinander jeweils ein linearer oder verzweigter $C_{1-6}$-Alkyl-Rest sind;
X -COO-, $-CON(R^6)$- oder entfällt, wobei die Bindung an A über O bzw. N erfolgt und wobei
$R^6$ H; oder ein aliphatischer linearer oder verzweigter $C_1$-$C_{20}$-Alkyl-Rest ist, der durch ein oder mehrere O oder S unterbrochen sein kann und der endständig eine polymerisationsfähige Vinyl-, (Meth)acryloyloxy-, (Meth)acrylamidgruppe, $-C(=CH_2)-COOR^3$ oder $-C(=CH_2)-CO-NR^4R^5$ tragen kann, wobei $R^{3-5}$ unabhängig voneinander jeweils ein linearer oder verzweigter $C_{1-6}$-Alkyl-Rest sind;
n eine ganze Zahl von 1 bis 6,

und/oder
einen Vinylsulfonester der Formel II

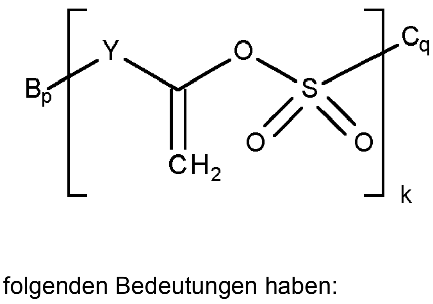

Formel II

,

in der die Variablen die folgenden Bedeutungen haben:

B H, CN, ein cycloaliphatischer, linearer oder verzweigter aliphatischer $C_1$-$C_{30}$-Kohlenwasserstoffrest, der durch

einen oder mehrere, vorzugsweise 1 bis 6, besonders bevorzugt 1 bis 3 Substituenten, vorzugsweise $-CH_3$, $-C_2H_5$, -OH, $-OCH_3$ und/oder $-O-COCH_3$, substituiert sein kann, der durch eine oder mehrere, vorzugsweise 1 bis 4, besonders bevorzugt 1 bis 2 Urethangruppen, Estergruppen, O und/oder S unterbrochen sein kann und der 1 bis 4, vorzugsweise 1 oder 2 Benzolgruppen enthalten kann, ein aromatischer $C_6$-$C_{30}$-Kohlenwasserstoffrest, der durch einen oder mehrere, vorzugsweise 1 bis 6, besonders bevorzugt 1 bis 3 Substituenten, vorzugsweise $-CH_3$, $-C_2H_5$, -OH, $-OCH_3$ und/oder $-O-COCH_3$, substituiert sein kann, oder eine Kombination davon;

Y -COO-, -CON($R^7$)- oder entfällt, wobei die Bindung an B über O oder N erfolgt;

C ein cycloaliphatischer, linearer oder verzweigter aliphatischer $C_1$-$C_{30}$-Kohlenwasserstoffrest, der durch einen oder mehrere, vorzugsweise 1 bis 6, besonders bevorzugt 1 bis 3 Substituenten, vorzugsweise $C_1$-$C_5$-Alkyl, -OH, -O-$COCH_3$ und/oder $C_1$-$C_5$-Alkoxy, substituiert sein kann, der durch eine oder mehrere, vorzugsweise 1 bis 4, besonders bevorzugt 1 bis 2 Urethangruppen, Estergruppen, O und/oder S unterbrochen sein kann und der 1 bis 4, vorzugsweise 1 oder 2 Benzolgruppen enthalten kann, ein aromatischer $C_6$-$C_{30}$-Kohlenwasserstoffrest, der durch einen oder mehrere, vorzugsweise 1 bis 6, besonders bevorzugt 1 bis 3 Substituenten, vorzugsweise Vinyl, $C_1$-$C_5$-Alkyl, -OH, $-O-COCH_3$ und/oder $C_1$-$C_5$-Alkoxy, substituiert sein kann, oder Tosyl, oder eine Kombination davon;

$R^7$ Wasserstoff, ein cycloaliphatischer, linearer oder verzweigter aliphatischer $C_1$-$C_{10}$-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann, oder ein aromatischer $C_6$-$C_{10}$-Kohlenwasserstoffrest, der durch eine oder mehrere, vorzugsweise 1 bis 4, besonders bevorzugt 1 bis 2 OH-Gruppen substituiert sein kann;

p eine ganze Zahl von 1 bis 6;

k eine ganze Zahl von 1 bis 6;

q eine ganze Zahl von 1 bis 6; wobei

p und q nicht gleichzeitig größer als 1 sein können und wobei dann, wenn p = 1 ist, q = k ist, und wenn q = 1 ist, p = k ist.

Bevorzugt sind Verbindungen der Formel I, in der die Variablen die folgenden Bedeutungen haben:

A H; ein aliphatischer linearer oder verzweigter $C_1$-$C_{14}$-Alkylen- oder $C_1$-$C_{14}$-Alkyl-Rest, der durch ein oder mehrere O oder S unterbrochen sein kann, der einen oder mehrere Substituenten, wie -OH oder $-OCH_3$, tragen kann und der endständig eine Tri-$C_1$-$C_3$-alkoxysilylgruppe oder eine polymerisationsfähige Vinyl-, (Meth)acryloyloxy- oder (Meth)acrylamidgruppe tragen kann;

$R^1$ H, ein aliphatischer verzweigter oder vorzugsweise linearer $C_1$-$C_4$-Alkyl-Rest, Tolyl oder Phenyl;

$R^2$ jeweils unabhängig voneinander ein Phenylrest, der einen oder mehrere Substituenten, wie $-CH_3$, $-C_2H_5$, -OH, $-OCH_3$, $-O-COCH_3$, tragen kann; oder ein aliphatischer linearer oder verzweigter $C_1$-$C_{10}$-Alkyl-Rest, der durch O oder S unterbrochen sein kann;

X -COO-, -CON($R^6$)- oder entfällt, wobei die Bindung an A über O bzw. N erfolgt und wobei

$R^6$ H; oder ein aliphatischer linearer oder verzweigter $C_1$-$C_{10}$-Alkyl-Rest ist, der durch ein oder mehrere O oder S unterbrochen sein kann;

n eine ganze Zahl von 1 bis 3.

Besonders bevorzugt sind Verbindungen der Formel I, in der die Variablen die folgenden Bedeutungen haben:

A H; ein aliphatischer linearer oder verzweigter $C_1$-$C_{12}$-Alkylen- oder $C_1$-$C_{12}$-Alkyl-Rest, der durch ein oder mehrere O unterbrochen sein kann und der endständig eine Tri-$C_1$-$C_2$-alkoxysilylgruppe oder eine polymerisationsfähige (Meth)acryloyloxygruppe tragen kann;

$R^1$ H oder ein aliphatischer linearer $C_1$-$C_4$-Alkyl-Rest;

$R^2$ jeweils unabhängig voneinander ein Phenylrest, der einen oder mehrere Substituenten, wie $-CH_3$, $-C_2H_5$, -OH, $-OCH_3$, tragen kann; oder ein aliphatischer linearer $C_1$-$C_6$-Alkyl-Rest, der durch O unterbrochen sein kann;

X -COO-, -CON($R^6$)- oder entfällt, wobei die Bindung an A über O bzw. N erfolgt und wobei

$R^6$ H; oder ein aliphatischer linearer $C_1$-$C_4$-Alkyl-Rest ist;

n eine ganze Zahl von 1 bis 2.

Bevorzugt sind Verbindungen der Formel II, in der die Variablen die folgenden Bedeutungen haben:

B H, ein cycloaliphatischer, linearer oder verzweigter aliphatischer $C_1$-$C_{15}$-Kohlenwasserstoffrest, der durch 1 bis 4, besonders bevorzugt 1 bis 3 Substituenten, vorzugsweise $-CH_3$, $-C_2H_5$, -OH, $-OCH_3$ und/oder $-O-COCH_3$, substituiert sein kann, der durch 1 bis 4, besonders bevorzugt 1 bis 2 Urethangruppen, Estergruppen, O und/oder S unterbrochen sein kann und der 1 oder 2 Benzolgruppen enthalten kann, ein aromatischer $C_6$-$C_{18}$-Kohlenwas-

serstoffrest, der durch 1 bis 6, besonders bevorzugt 1 bis 3 Substituenten, vorzugsweise $-CH_3$, $-C_2H_5$, -OH, $-OCH_3$ und/oder $-O-COCH_3$, substituiert sein kann, oder eine Kombination davon;

Y -COO-, $-CON(R^7)-$ oder entfällt, wobei die Bindung an B über O oder N erfolgt;

C ein cycloaliphatischer, linearer oder verzweigter aliphatischer $C_1$-$C_{15}$-Kohlenwasserstoffrest, der durch 1 bis 4, besonders bevorzugt 1 bis 3 Substituenten, vorzugsweise $C_1$-$C_4$-Alkyl, -OH, $-O-COCH_3$ und/oder $C_1$-$C_4$-Alkoxy, substituiert sein kann, der durch 1 bis 4, besonders bevorzugt 1 bis 2 Urethangruppen, Estergruppen, O und/oder S unterbrochen sein kann und der 1 oder 2 Benzolgruppen enthalten kann, ein aromatischer $C_6$-$C_{18}$-Kohlenwasserstoffrest, der durch 1 bis 6, besonders bevorzugt 1 bis 3 Substituenten, vorzugsweise Vinyl, $C_1$-$C_4$-Alkyl, -OH, $-O-COCH_3$ und/oder $C_1$-$C_4$-Alkoxy, substituiert sein kann, oder Tosyl, oder eine Kombination davon;

$R^7$ Wasserstoff ein cycloaliphatischer, linearer oder verzweigter aliphatischer $C_1$-$C_6$-Kohlenwasserstoffrest, der durch eine oder mehrere, vorzugsweise 1 bis 2 Sauerstoffatome unterbrochen sein kann und der durch eine oder mehrere, vorzugsweise 1 bis 2 OH-Gruppen substituiert sein kann, oder ein aromatischer $C_6$-$C_{10}$-Kohlenwasserstoffrest, der durch eine oder mehrere, vorzugsweise 1 bis 2 OH-Gruppen substituiert sein kann;

p eine ganze Zahl von 1 bis 3;

k eine ganze Zahl von 1 bis 3;

q eine ganze Zahl von 1 bis 3; wobei

p und q nicht gleichzeitig größer als 1 sein können und wobei dann, wenn p = 1 ist, q = k ist, und wenn q = 1 ist, p = k ist.

Besonders bevorzugt sind Verbindungen der Formel II, in der die Variablen die folgenden Bedeutungen haben:

B H, ein cycloaliphatischer, linearer oder verzweigter aliphatischer $C_1$-$C_{10}$-Kohlenwasserstoffrest, der durch 1 bis 3 Substituenten, vorzugsweise $-CH_3$, -OH, und/oder $-OCH_3$, substituiert sein kann und der durch 1 bis 2 O unterbrochen sein kann, ein aromatischer $C_6$-$C_{10}$-Kohlenwasserstoffrest, der durch 1 bis 3 Substituenten, vorzugsweise $-CH_3$, -OH, und/oder $-OCH_3$, substituiert sein kann, oder eine Kombination davon;

Y -COO-, wobei die Bindung an B über O erfolgt;

C ein cycloaliphatischer, linearer oder verzweigter aliphatischer $C_1$-$C_{10}$-Kohlenwasserstoffrest, der durch 1 bis 3 Substituenten, vorzugsweise $C_1$-$C_3$-Alkyl, -OH, $-O-COCH_3$ und/oder $C_1$-$C_3$-Alkoxy, substituiert sein kann, der durch 1 bis 2 O und/oder S unterbrochen sein kann und der 1 oder 2 Benzolgruppen enthalten kann, ein aromatischer $C_6$-$C_{10}$-Kohlenwasserstoffrest, der durch 1 bis 3 Substituenten, vorzugsweise Vinyl, $C_1$- bis $C_3$-Alkyl, -OH, $-O-COCH_3$ und/oder $C_1$- bis $C_3$-Alkoxy, substituiert sein kann, oder Tosyl, oder eine Kombination davon;

p eine ganze Zahl von 1 bis 2;

k eine ganze Zahl von 1 bis 2;

q eine ganze Zahl von 1 bis 2; wobei

p und q nicht gleichzeitig größer als 1 sein können und wobei dann, wenn p = 1 ist, q = k ist, und wenn q = 1 ist, p = k ist.

Die Formeln erstrecken sich nur auf solche Verbindungen, die mit der chemischen Valenzlehre vereinbar sind. Beispielsweise kann n dann, wenn A Wasserstoff ist, nur 1 sein, und p kann dann, wenn B ein $C_1$-Rest ist, maximal 4 sein. Der Hinweis, dass ein Rest durch eine oder mehrere Urethangruppen, O-Atome, S-Atome etc. unterbrochen ist, ist so zu verstehen, dass diese Gruppen jeweils in die Kohlenstoffkette des Restes eingeschoben werden. Diese Gruppen sind damit beidseitig durch C-Atome begrenzt und können nicht endständig sein. $C_1$-Reste können nicht unterbrochen sein. Der Hinweis, dass ein Rest eine Benzolgruppe enthält, bedeutet demgegenüber, dass diese Gruppe auch endständig sein kann, wobei ggf. verbleibende yl-Stellen durch H abgesättigt sind. Unter Kombinationen werden Gruppen verstanden, die sich aus den jeweils angegebenen Bedeutungen zusammensetzen, beispielsweise aus aromatischen und aliphatischen Resten, wie z.B. $-Ph-CH_2-Ph-$, oder aus mehreren aromatischen Resten, wie z.B. -Ph-Ph-, oder aus aromatischen und/oder aliphatischen Resten und anderen der genannten Gruppen, wie z.B. -Ph-O-Ph- (Ph=Phenyl).

Geeignete Allylsulfone der Formel I und ihre Herstellung sind in der EP 2 965 741 A1 beschrieben. Besonders geeignete Allylsulfone der Formel I sind 2-(Toluol-4-sulfonylmethyl)-acrylsäureethylester (TSMEA), 2-(Toluol-4-sulfonylmethyl)-acrylsäure-2-(2-ethoxyethoxy)-ethylester, 2-(Toluol-4-sulfonylmethyl)-acrylsäure, 2-(Toluol-4-sulfonylmethyl)-acrylsäure-tert-butylester, 3-(Trimethoxysilyl)propyl-2-(tosylmethyl)-acrylat, 3-(Triethoxysilyl)propyl-2-(tosylmethyl)acrylat, 3-(Triethoxysilyl)propyl-2-(tosylmethyl)acrylamid, 2-(Methylsulfonylmethyl)-ethylacrylat, 2-(Methylsulfonylmethyl)-propylacrylat, Triethylenglycol-bis[2-(toluol-4-sulfonylmethyl)-acrylat], 2-(Toluol-4-sulfonylmethyl)-acrylsäure-(2-methacryloyloxyethyl)ester, 2-(Toluol-4-sulfonylmethyl)-acrylsäure-(6-methacryloyloxyhexyl)ester, 2-(Toluol-4-sulfonylmethyl)-acrylsäure-(10-methacryloyloxydecyl)ester, 2-(Toluol-4-sulfonylmethyl)-acrylsäure-(2-hydroxy-3-methacryloyloxypropyl)ester und 2-(Toluol-4-sulfonylmethyl)-acrylsäure-(8-methacryloyloxy-3,6-dioxaoctyl)ester, wobei 2-(Toluol-4-sulfonylmethyl)-acrylsäureethylester (TSMEA) ganz besonders geeignet ist:

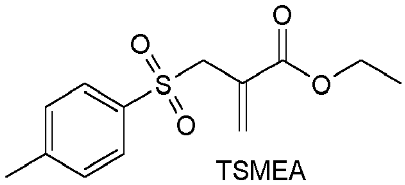

TSMEA

Geeignete Vinylsulfonester der Formel II und ihre Herstellung sind in der EP 3 090 772 A1 beschrieben. Besonders geeignete Vinylsulfonester der Formel II sind 2-(Toluol-4-sulfonyloxy)-acrylsäureethylester (TSVEA), 2-Methansulfonyloxyacrylsäureethylester, Triethylenglykol-bis[2-(toluol-4-sulfonyloxy)-acrylat], 2-(2,4,6-Triisopropylbenzolsulfonyloxy-acrylsäureoctylester, 2-(Toluol-4-sulfonyloxy)-acrylsäure-2-(tetrahydropyran-2-yloxy)-ethylester, 2-(Toluol-4-sulfonyloxy)-acrylsäure-2-hydroxyethylester, 2-(Toluol-4-sulfonyloxy)-acrylsäure-2,4,6-trimethylphenylester, 2-(4-Vinylbenzolsulfonyloxy)-acrylsäureethylester, 2-(Toluol-4-sulfonyloxy)-acrylsäuremethoxymethylester, 2-(2,4,6-Triisopropylbenzolsulfonyloxy)-acrylsäuremethoxymethylester, 2-(2,4,6-Triisopropylbenzolsulfonyloxy)-acrylsäure und Diethyl-2,2'-[(1,3-phenyldisulfonyl)bis(oxy)]-diacrylat, wobei 2-(Toluol-4-sulfonyloxy)-acrylsäureethylester (TSVEA) ganz besonders geeignet ist:

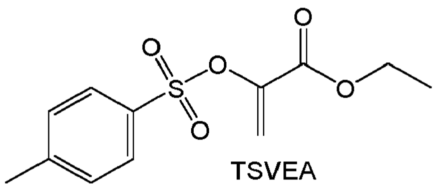

TSVEA

Erfindungsgemäß bevorzugt sind Werkstoffe, die als radikalisch polymerisierbares Monomer (b) mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Die mono- oder multifunktionellen (Meth)acrylate können weitere funktionelle Gruppen, wie z.B. Hydroxy-, Ester-Silyl- oder Ureido-Gruppen enthalten. Über die Struktur der radikalisch polymerisierbaren (Meth)acrylat-Comonomere lassen sich die Flexibilität und die Eigenschaften der gehärteten Materialien gezielt beeinflussen. So führt z.B. ein höherer Anteil von multifunktionellen (Meth)acrylaten zu einem höheren Vernetzungsgrad und damit zu einem Material mit geringerer Flexibilität. Dem entgegen führt die Zugabe von z.B. monofunktionellen (Meth)acrlyaten mit beweglichen Seitenketten zu einer höheren Flexibilität. In den (Meth)acrylaten enthaltene funktionelle Gruppen, wie z.B. Hydroxy-Gruppen können durch polare Wechselwirkungen die Festigkeit des Materials erhöhen.

Geeignete mono- oder multifunktionelle (Meth)acrylate sind 2-Hydroxyethyl-, Benzyl-, Tetrahydrofurfuryl- oder Isobornylmethacrylat, p-Cumyl-phenoxyethylenglycolmethacrylat (CMP-1E), Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylat, wie z.B. 2-[4-(2-(Meth)acryloyloxyethoxyethoxy)phenyl]-2-[4-(2-methacryloyloxyethoxy)phenyl]-propan) (SR-348c, Firma Sartomer; enthält 3 Ethoxygruppen) oder 2,2-Bis[4-(2-methacryloxypropoxy)-phenyl]propan (Bis-PMA), UDMA (ein Additionsprodukt aus 2-Hydroxyethylmethacrylat und 2,2,4- oder 2,4,4-Trimethylhexa-methylen-1,6-diisocyanat), Di-, Tri- oder Tetraethylenglycoldimethacrylat, Trimethylolpropantrimethacrylat, Pentaerythrittetramethacrylat, sowie Glycerindi- und trimethacrylat, Bismethacryloyloxymethyltricyclo[5.2.1.]decan (TCDMA), 1,4-Butandioldimethacrylat, 1,10-Decandioldimethacrylat ($D_3MA$), 1,12-Dodecandioldimethacrylat oder Mischungen davon.

Besonders geeignet sind Bisphenol-A-dimethacrylat, Bis-GMA (ein Additionsprodukt aus Methacrylsäure und Bisphenol-A-di-glycidylether), 2-[4-(2-(Meth)acryloyloxyethoxyethoxy)phenyl]-2-[4-(2-methacryloyloxyethoxy)phenyl]-propan) (SR-348c, Sartomer), 2,2-Bis[4-(2-methacryloxypropoxy)phenyl]propan (Bis-PMA), UDMA (ein Additionsprodukt aus 2-Hydroxyethylmethacrylat und 2,2,4- oder 2,4,4-Trimethylhexamethylen-1,6-diisocyanat), Bismethacryloyloxymethyltricyclo[5.2.1.]decan (TCDMA) und Mischungen davon.

Die genannten Monomere eignen sich besonders zur Herstellung von Dentalwerkstoffen.

Die erfindungsgemäßen Werkstoffe enthalten als Photoinitiatoren (c) vorzugsweise eine Kombination mindestens eines monomolekularen Photoinitiators und mindestens eines bimolekularen Photoinitiators, insbesondere solche Photoinitiatoren, die in einem Wellenlängenbereich von 400 bis 500 nm aktiv sind. Bevorzugt werden der mindestens eine monomolekulare Photoinitiator und der mindestens eine bimolekulare Photoinitiator in einem Verhältnis von 2:1 bis 1:2, bezogen auf ihre Gewichtsanteile, eingesetzt. Besonders bevorzugt werden der mindestens eine monomolekulare Photoinitiator und der mindestens eine bimolekulare Photoinitiator in einem Verhältnis von 1,4:1 bis 1:2, bezogen auf ihre Gewichtsanteile, eingesetzt. Ganz besonders bevorzugt werden der mindestens eine monomolekulare Photoinitiator und der mindestens eine bimolekulare Photoinitiator in einem Verhältnis von 1,2:1 bis 1:1,8, und am meisten

bevorzugt 1:1 bis 1:1,8, bezogen auf ihre Gewichtsanteile, eingesetzt.

Bevorzugte monomolekulare Photoinitiatoren sind Monoacyl- oder Bisacylphosphinoxide, Diacyldialkylgermanium- und Tetraacylgermanium-Verbindungen sowie Tetraacylstannane. Besonders bevorzugte monomolekulare Photoinitiatoren sind 2,4,6-Trimethylbenzoyldiphenylphosphinoxid, Bis(2,4,6-trimethylbenzoyl)-phenylphosphinoxid, Dibenzoyldiethylgerman, Bis(4-methoxybenzoyl)diethylgerman (MBDEGe, Ivocerin®), Tetrabenzoylgerman, Tetrakis(o-methylbenzoyl)german, Tetrakis(mesitoyl)stannan und Mischungen davon.

Als bimolekulare Photoinitiatoren eigen sich vorzugsweise $\alpha$-Diketone oder deren Derivate. Besonders geeignete $\alpha$-Diketone oder Derivate davon sind Campherchinon (CQ), 9,10-Phenanthrenchinon, 1-Phenyl-propan-1,2-dion, 2,2-Dimethoxy-2-phenyl-acetophenon, Diacetyl oder 4,4'-Dichlorbenzil oder deren Derivate. Ganz besonders bevorzugt sind Campherchinon (CQ), 2,2-Dimethoxy-2-phenyl-acetophenon und Mischungen davon. Am meisten bevorzugt sind $\alpha$-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin, Triethanolamin und Mischungen davon. Vorzugsweise wird ein Verhältnis von bimolekularem Photoinitiator zu Amin von 1:1 bis 1:6, bezogen auf ihre Gewichtsanteile, eingesetzt.

Besonders bevorzugte Kombinationen mindestens eines monomolekularen Photoinitiators, mindestens eines bimolekularen Photoinitiators und mindestens eines Amins als Reduktionsmittel sind Mischungen von mindestens einem aus Bis(4-methoxybenzoyl)diethylgerman (MBDEGe, Ivocerin®), Bis(2,4,6-trimethylbenzoyl)phenylphosphinoxid, Tetrakis(o-methylbenzoyl)german und Tetrakis(mesitoyl)stannan als monomolekularem Photoinitiator, Campherchinon (CQ) und/oder 2,2-Dimethoxy-2-phenyl-acetophenon als bimolekularem Photoinitiator und 4-(Dimethyl-amino)benzoesäureester (EDMAB) als Reduktionsmittel.

Ganz besonders bevorzugte Kombinationen eines monomolekularen Photoinitiators, mindestens eines bimolekularen Photoinitiators und eines Amins als Reduktionsmittel sind Mischungen von Bis(4-methoxybenzoyl)diethylgerman (MBDEGe, Ivocerin®) als monomolekularem Photoinitiator, Campherchinon (CQ) und/oder 2,2-Dimethoxy-2-phenyl-acetophenon als bimolekularem Initiator und 4-(Dimethylamino)benzoesäureester (EDMAB) als Reduktionsmittel.

Bevorzugte Füllstoffe (d) sind anorganische partikuläre Füllstoffe, insbesondere Oxide, wie $SiO_2$, $ZrO_2$ und $TiO_2$ oder Mischoxide aus $SiO_2$, $ZrO_2$, ZnO und/oder $TiO_2$, nanopartikuläre oder mikrofeine Füllstoffe, wie pyrogene Kieselsäure oder Fällungskieselsäure, Glaspulver, wie Quarz-, Glaskeramik- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von $SiO_2$ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten.

Bevorzugt weisen die Oxide, wie $SiO_2$, $ZrO_2$ und $TiO_2$ oder Mischoxide aus $SiO_2$, $ZrO_2$, ZnO und/oder $TiO_2$ eine Partikelgröße von 0,010 bis 15 $\mu$m, die nanopartikulären oder mikrofeinen Füllstoffe, wie pyrogene Kieselsäure oder Fällungskieselsäure, eine Partikelgröße von 10 bis 300 nm, die Glaspulver, wie Quarz-, Glaskeramik- oder röntgenopake Glaspulver, vorzugsweise Barium- oder Strontiumaluminiumsilikatgläser, eine Partikelgröße von 0,01 bis 15 $\mu$m, vorzugweise von 0,2 bis 1,5 $\mu$m, und die röntgenopaken Füllstoffe, wie Ytterbiumtrifluorid, Tantal(V)-oxid, Bariumsulfat oder Mischoxide von $SiO_2$ mit Ytterbium(III)-oxid oder Tantal(V)-oxid eine Partikelgröße von 0,2 bis 5 $\mu$m auf.

Besonders bevorzugte Füllstoffe sind Mischoxide aus $SiO_2$ und $ZrO_2$, mit einer Partikelgröße von 10 bis 300 nm, Glaspulver mit einer Partikelgröße von 0,2 bis 1,5 $\mu$m, wie röntgenopake Glaspulver von z.B. Barium- oder Strontiumaluminiumsilikatgläsern, und röntgenopake Füllstoffe mit einer Partikelgröße von 0,2 bis 5 $\mu$m, wie Ytterbiumtrifluorid, oder Mischoxide von $SiO_2$ mit Ytterbium(III)-oxid.

Außerdem sind als Füllstoff gemahlene Präpolymerisate (Isofüller) geeignet, die eine Mischung von Polymerpartikeln sowie röntgenopakem/n Glaspulver(n) und Ytterbiumtrifluorid enthalten.

Wenn nicht anders angegeben, handelt es sich bei allen Partikelgrößen um gewichtsmittlere Partikelgrößen, bei denen die Partikelgrößenbestimmung im Bereich von 0,1 $\mu$m bis 1000 $\mu$m mittels statischer Lichtstreuung erfolgt, vorzugsweise mit einem statischen Laserstreuungs-Partikelgrößen-Analysator LA-960 (Horiba, Japan). Hierbei werden als Lichtquellen eine LaserDiode mit einer Wellenlänge von 655 nm und eine LED mit einer Wellenlänge von 405 nm in einem Messbereich von 0,1 bis 1000 $\mu$m verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 $\mu$m werden vorzugsweise mittels Dynamischer Lichtstreuung (DLS) bestimmt. Die Messung der Partikelgröße im Bereich von 5 nm bis 0,1 $\mu$m erfolgt vorzugsweise durch Dynamische Lichtstreuung (DLS) von wässrigen Partikeldispersionen, vorzugsweise mit einem Malvern zetasizer nano ZS (Malvern Instruments, Malvern UK) mit einem He-Ne-Laser mit einer Wellenlänge von 633 nm, bei einem Streuwinkel von 90° bei 25°C.

Partikelgrößen kleiner als 0,1 $\mu$m können auch mittels REM- oder TEM-Spektroskopie bestimmt werden. Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem

Kupfergitter (Maschenweite 300) aufgebracht, welches mit Kohlenstoff beschichtet ist, und im Anschluss das Lösungsmittel verdampft.

Die Lichtstreuung nimmt mit abnehmender Partikelgröße ab, jedoch haben Füllstoffe mit geringer Partikelgröße eine größere Verdickungswirkung. Die Füllstoffe werden nach der Partikelgröße unterteilt in Makrofüller und Mikrofüller. Makrofüller werden durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 10 µm. Als Mikrofüller werden vorzugsweise pyrogenes $SiO_2$ oder Fällungskieselsäure eingesetzt, oder auch Mischoxide, z.B. $SiO_2$-$ZrO_2$, die durch hydrolytische Cokondensation von Metallalkoxiden zugänglich sind. Die Mikrofüller haben vorzugsweise eine mittlere Partikelgröße von ca. 5 bis 100 nm.

Die Füllstoffe sind vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von $ZrO_2$ oder $TiO_2$, können auch funktionalisierte saure Phosphate, wie z.B. 10-Methacryloyloxydecyldihydrogenphosphat eingesetzt werden.

Die erfindungsgemäßen Werkstoffe können gegebenenfalls weitere Additive enthalten, vor allem Stabilisatoren, Farbmittel, mikrobiozide Wirkstoffe, fluoridionenabgebende Additive, Treibmittel, optische Aufheller, Weichmacher oder UV-Absorber. Erfindungsgemäß wurde gefunden, dass durch die Kombination von bestimmten Übertragungsreagenzien, radikalisch polymerisierbaren Monomeren und Photoinitiatoren Werkstoffe erhalten werden können, die bereits nach einer kurzen Belichtungszeit aushärten. Die erfindungsgemäßen Werkstoffe lassen sich beispielsweise durch Belichtung für 10 s mit einer Strahldichte von 1000 bis 1400 mW/$cm^2$ härten. Vorzugsweise erfolgt die Härtung durch Belichtung für 5 s mit einer Strahldichte von 1600 bis 2400 mW/$cm^2$, ganz besonders bevorzugt durch Belichtung für 1 s mit einer Strahldichte von 8000 bis 12000 mW/$cm^2$ und am meisten bevorzugt durch Belichtung für 3 s mit einer Strahldichte von 2700 bis 3500 mW/$cm^2$ oder für 2 s mit einer Strahldichte von 4000 bis 6000 mW/$cm^2$. Die erfindungsgemäßen Werkstoffe haben den Vorteil, dass sich trotz der kurzen Belichtungszeit und der damit verbundenden kurzen Aushärtezeit nur eine geringe PSK aufbaut, sodass sich randspaltfreie Füllungen ergeben. Die Werkstoffe eigenen sich daher besonders als Dentalkomposite.

Die erfindungsgemäßen Werkstoffe weisen vorzugsweise die folgende Zusammensetzung auf:

(a) 0,1 bis 5 Gew.-%, bevorzugt 0,1 bis 3 Gew.-% mindestens eines Übertragungsreagenzes der Formel(n) I und/oder II,
(b) 8 bis 50 Gew.-%, bevorzugt 10 bis 40 Gew.-% mindestens eines multifunktionellen (Meth)acrylates oder einer Mischung von mono- und multifunktionellen (Meth)acrylaten,
(c) 0,05 bis 3,0 Gew.-% und bevorzugt 0,1 bis 2,0 Gew.-% einer Mischung mindestens eines monomolekularen und mindestens eines bimolekularen Photoinitiators, wobei der mindestens eine monomolekulare Photoinitiator und der mindestens eine bimolekulare Photoinitiator vorzugsweise in einem Verhältnis von 1,4:1 bis 1:2, besonders bevorzugt in einem Verhältnis von 1,2:1 bis 1:2, bezogen auf ihre Gewichtsanteile, eingesetzt werden,
(d) 40 bis 90 Gew.-% und bevorzugt 50 bis 85 Gew.-% mindestens eines Füllstoffs, und
(e) 0 bis 5 Gew.-%, bevorzugt 0 bis 3 Gew.-% und besonders bevorzugt 0,2 bis 3 Gew.-% Additiv(e).

Die erfindungsgemäßen Werkstoffe enthalten besonders bevorzugt die folgenden Komponenten:

(a) 0,1 bis 5 Gew.-%, bevorzugt 0,1 bis 3 Gew.-% mindestens eines Übertragungsreagenzes der Formel(n) I und/oder II,
(b) 10 bis 40 Gew.-% mindestens eines multifunktionellen (Meth)acrylates ausgewählt aus Bisphenol-A-dimethacrylat, Bis-GMA (ein Additionsprodukt aus Methacrylsäure und Bisphenol-A-diglycidylether), 2-[4-(2-(Meth)acryloyloxyethoxyethoxy)phenyl]-2-[4-(2-methacryloyloxyethoxy)phenyl]-propan) (SR-348c, Sartomer), 2,2-Bis[4-(2-methacryloxy-propoxy)phenyl]propan (Bis-PMA), UDMA (ein Additionsprodukt aus 2-Hydroxyethylmethacrylat und 2,2,4- oder 2,4,4-Tri-methylhexamethylen-1,6-diisocyanat), Bismethacryloyloxy-methyltricyclo[5.2.1.]decan (TCDMA) und Mischungen davon,
(c) 0,1 bis 2,0 Gew.-% einer Mischung von Diacyldialkylgermanium-, Tetraacylgermanium- und Tetraacylstannan-Verbindungen oder Mono- oder Bisacylphosphinoxiden mit einem bimolekularen Photoinitiator, vorzugsweise Campherchinon (CQ) und/oder 2,2-Dimethoxy-2-phenyl-acetophenon, sowie mit 4-(Dimethylamino)benzoesäureester (EDMAB), wobei der mindestens eine monomolekulare Photoinitiator und der mindestens eine bimolekulare Photoinitiator vorzugsweise in einem Verhältnis von 1,4:1 bis 1:2, besonders bevorzugt in einem Verhältnis von 1,2:1 bis 1:2, bezogen auf ihre Gewichtsanteile, eingesetzt werden,
(d) 50 bis 85 Gew.-% Füllstoff ausgewählt aus Mischoxiden aus $SiO_2$, $ZrO_2$, Glaspulver, wie röntgenopakem Glaspulver von z.B. Barium- oder Strontiumaluminiumsilikatgläsern, und röntgenopaken Füllstoffen, wie Ytterbiumtrifluorid, oder Mischoxiden von $SiO_2$ mit Ytterbium(III)-oxid, gemahlenen Präpolymerisaten, die eine Mischung von Polymerpartikeln sowie röntgenopakem/n Glaspulver(n) und Ytterbiumtrifluorid enthalten, und Mischungen davon,

und
(f) 0,2 bis 3 Gew.-% Additiv(e).

Alle Prozentangaben hierin sind in Gewichtsprozent und beziehen sich auf die Gesamtmasse des Werkstoffs, wenn nicht anders angegeben.

Besonders bevorzugt sind Werkstoffe, die aus den genannten Stoffen bestehen. Weiterhin sind solche Werkstoffe bevorzugt, bei denen die einzelnen Komponenten jeweils aus den oben genannten bevorzugten und besonders bevorzugten Stoffen ausgewählt sind.

Die erfindungsgemäßen Werkstoffe weisen zudem mechanische Eigenschaften auf, wie z.B. eine hohe Biegefestigkeit, ein hohes Biegemodul, eine große Härte und eine geringe Schrumpfspannung, die für dentale Anwendungen vorteilhaft sind, insbesondere für Prothesen oder stereolithographisch hergestellte Formkörper. Sie erlauben die Herstellung von Prothesen und Formkörpern mit großer Dimensionsstabilität und hoher Bruchfestigkeit.

Ein wesentlicher Vorteil der erfindungsgemäßen Werkstoffe ist ihre geringere Schrumpfungsspannung. Die Werkstoffe haben nach der Polymerisation bevorzugt eine Schrumpfungsspannung von 60 bis 110 N, besonders bevorzugt von 65 bis 100 N und ganz besonders bevorzugt von 70 bis 90 N. Die Schrumpfungsspannung kann z.B. mit einem Bioman Schrumpfungsspannungsmessgerät bestimmt werden. Hierfür wird eine definierte Menge des Materials in einen Spalt zwischen einen Metallzylinder, der an einem Kraftarm befestigt ist, und einer Glasplatte eingebracht und anschliessend durch Belichtung durch die Glasplatte hindurch gehärtet (1, 2, 3 und 10 s bei 10000, 5000, 3400 bzw. 1200 $mW/cm^2$). Der dadurch auf den Kraftarm aufgebrachte Zug wird mit einem verstärkten Spannungsanzeige-Energieumwandler in ein elektrisches Signal umgewandelt und aufgezeichnet. Anhand einer Kalibrierungsgeraden werden die elektrischen Signale in die Schrumpfungsspannung in Newton (N) umgerechnet. Durch Division der Schrumpfungsspannung durch die Kontaktfläche des Messzylinders erhält man den Schrumpfungsstress in N. Weiterhin weisen die erfindungsgemäßen Werkstoffe den Vorteil einer hohen Biegefestigkeit auf. Die erfindungsgemäßen Werkstoffe weisen nach der Polymerisation bevorzugt eine Biegefestigkeit von 75 bis 140 MPa, besonders bevorzugt von 80 bis 135 MPa und ganz besonders bevorzugt von 85 bis 130 MPa auf. Die Bestimmung der Biegefestigkeit erfolgt nach der ISO-Norm ISO-4049 (Dentistry - Polymer-based filling, restorative and luting materials) .

Außerdem zeichnen sich die erfindungsgemäßen Werkstoffe durch ein hohes Biegemodul aus. Die Werkstoffe weisen nach der Polymerisation bevorzugt ein Biegemodul von 8,5 bis 12,5 GPa, besonders bevorzugt von 9,0 bis 12,0 GPa und ganz besonders bevorzugt von 9,3 bis 11,8 GPa auf. Die Bestimmung des Biege-E-Moduls erfolgt nach der ISO-Norm ISO-4049 (Dentistry - Polymer-based filling, restorative and luting materials).

Ein weiterer Vorteil der erfindungsgemäßen Werkstoffe ist Ihre große Härte. Die Werkstoffe haben nach der Polymerisation bevorzugt eine Vickers-Härte von 500 bis 700 MPa, besonders bevorzugt von 500 bis 670 MPa und ganz besonders bevorzugt von 500 bis 650 MPa. Die Härteprüfung nach Vickers erfolgt in 4 mm Tiefe durch Messung des Härteprofils (VH) nach EN ISO 6507-1:2006-03. Die Vickershärte wird vorzugsweise mit einer normierten Härte-Prüfmaschine gemessen (Modell ZHU0.2/Z2.5, Fa. Zwick Roell, Eindringkörper: Vickers-Diamantpyramide (136°), variabler Härtemesskopf, Prüfkraftbereich von 2 bis 200 Newton) .

Die erfindungsgemäßen Werkstoffe eigenen sich besonders als dentale Füllungsmaterialien, Zemente und Verblendmaterialien sowie als Materialien zur Herstellung von Prothesen, künstlichen Zähnen, Inlays, Onlays, Kronen und Brücken.

Die Dentalwerkstoffe eignen sich primär zur intraoralen Anwendung durch den Zahnarzt zur Restauration geschädigter Zähne (klinische Materialien), d.h. zur therapeutischen Anwendung, z.B. als dentale Zemente, Füllungskomposite und Verblendmaterialien. Sie können aber auch extraoral, d.h. nicht therapeutisch, eingesetzt werden, beispielweise bei der Herstellung oder Reparatur von Dentalrestaurationen, wie Prothesen, künstlichen Zähnen, Inlays, Onlays, Kronen und Brücken (technische Materialien).

Die erfindungsgemäßen Werkstoffe eigenen sich außerdem zur Herstellung von Formkörpern für dentale aber auch für nicht dentale Zwecke, z.B. mittels Gießen, Pressen und insbesondere durch generative Verfahren wie den 3D-Druck.

Die Erfindung wird im Folgenden anhand von Beispielen näher erläutert.

## Ausführungsbeispiele

### Beispiel 1:

*Herstellung von Kompositen auf Basis von Allyl- bzw. Vinylsulfonestern*

Kompositpasten mit den Zusammensetzungen gemäß Tabelle 1 wurden mittels Kneter hergestellt. Alle Kompositpasten enthielten eine Mischung von Bis-GMA, UDMA, SR-348c, Bis-PMA und TCDMA. Als Photoinitiatormischung enthielten sie zudem einen monomolekularen Initiator (Bis(4-methoxybenzoyl)diethylgerman, Ivocerin®), einen bimolekularen Initiator (Campherchinon (CQ)) und ein Amin als Reduktionsmittel (4-(Dimethylamino)-benzoesäureester (EDMAB)). Das Kompositmaterial B enthielt als Übertragungsreagenz einen Allylsulfonester **(TSMEA),** das Material C einen Vinylsulfonester **(TSVEA).** Das Vergleichsmaterial A enthielt kein Übertragungsreagenz.

Aus den Kompositpasten wurden entsprechende Prüfkörper präpariert und jeweils mit einer Polymerisationslampe (Bluephase G4 (390-510 nm) mit Power Cure (3s) Modus) für 10 s (1200 $mW/cm^2$), 3 s (3060 $mW/cm^2$), 2 s (5000 $mW/cm^2$) und 1 s (10000 $mW/cm^2$) belichtet und damit ausgehärtet. Die Bestimmung der Biegefestigkeit und des Biege-E-Moduls erfolgte nach der ISO-Norm ISO-4049 (Dentistry - Polymer-based filling, restorative and luting materials) (Tabelle 2). Die Messungen wurden nach 24 h Lagerung in Wasser (37 °C) durchgeführt.

Der Doppelbindungsumsatz in 4 mm Tiefe (DBU) wurde mittels FTIR-Spektroskopie (Vertex 70 IR-Spektrometer, Bruker) bestimmt. Um den DBU zu berechnen, wurden die Peaks der aliphatischen (ca. 1650-1625 $cm^{-1}$) und aromatischen (ca. 1620-1600 $cm^{-1}$) Doppelbindungen in den gemessenen Spektren integriert und aus den zuvor bestimmten Integralen für jedes gemessene Spektrum das Verhältnis der aliphatischen zu den aromatischen Doppelbindungen berechnet. Anschließend wurde für die Verhältnisse (V) der unpolymerisierten Proben und der polymerisierten Proben jeweils der Mittelwert (MW) dieser Verhältnisse berechnet. Abschließend wurden aus den berechneten Mittelwerten gemäß der folgenden Formel die Werte für den DBU bestimmt:

$$\boldsymbol{DBU}[\%] = (\mathbf{1} - \boldsymbol{MW}\ \boldsymbol{V}_{\mathrm{polymerisiert}}/\boldsymbol{MW}\ \boldsymbol{V}_{\mathrm{unpolymerisiert}}) * \mathbf{100}.$$

Die Härte in 4 mm Tiefe wurde mittels Vickershärteprofil-Messungen (VH) bestimmt. Die Härteprüfung nach Vickers erfolgte nach EN ISO 6507-1:2006-03 mit einer normierten Härte-Prüfmaschine (Modell ZHU0.2/Z2.5, Fa. Zwick Roell, Eindringkörper: Vickers-Diamantpyramide (136°), variabler Härtemesskopf, Prüfkraftbereich von 2 bis 200 Newton).

Die Schrumpfungsspannung (SK) der Komposite wurde mit einem Bioman Schrumpfungsspannungsmessgerät bestimmt. Hierfür wurde eine definierte Kompositmenge von 0,15 bis 0,25 g in einen Spalt zwischen einen Metallzylinder, der an einem Kraftarm befestigt ist, und einer Glasplatte eingebracht und anschließend durch die Glasplatte hindurch mit einer dentalen Belichtungseinheit bestrahlt und dadurch polymerisiert. Der entstehende Zug auf den Kraftarm wurde mit einem verstärkten Spannungsanzeige-Energieumwandler in ein elektrisches Signal umgewandelt und aufgezeichnet. Anhand einer Kalibriergeraden wurden die elektrischen Signale in die Schrumpfungsspannung in Newton (N) umgerechnet.

Der Komposit A (ohne Übertragungsreagenz) hat im Vergleich zu den Kompositen B (TSMEA) und C (TSVEA) bei Belichtungszeiten unter 10 s schlechtere mechanische Eigenschaften. Trotz des höheren Doppelbindungsumsatzes fallen die Werte für die Schrumpfkraft bei den Kompositen B und C im Vergleich zu Komposit A aber deutlich niedriger aus.

***Tabelle 1: Zusammensetzung der Komposite***

| Funktion | Komponente | Komposit A* (Gew.-%) | Komposit B (Gew.-%) | Komposit C (Gew.-%) |
|---|---|---|---|---|
| Monomer | Bis-GMA[a] | 10,0 | 9,1 | 9,1 |
| | UDMA[b] | 5,0 | 5,0 | 5,0 |
| | SR-348c[c] | 2,3 | 2,3 | 2,3 |
| | Bis-PMA[d] | 3,5 | 3,5 | 3,5 |
| | TCDMA[e] | 1 | 1 | 1 |
| Initiator | Ivocerin®[f] | 0,03 | 0,03 | 0,03 |
| | Campherchinon | 0,04 | 0,04 | 0,04 |
| | EDMAB[g] | 0,13 | 0,13 | 0,13 |
| Füllstoff | Isofüller[h] | 18 | 18 | 18 |
| | Glasfüller[i] | 43 | 43 | 43 |
| | $SiO_2$-$ZrO_2$ Sphärosil[j] | 11 | 11 | 11 |
| | $YbF_3$[k] | 6 | 6 | 6 |

(fortgesetzt)

| Funktion | Komponente | Komposit A* (Gew.-%) | Komposit B (Gew.-%) | Komposit C (Gew.-%) |
|---|---|---|---|---|
| Übertragungsreagenz | TSVEA[l] | - | 0,9 | - |
| | TSMEA[m] | - | | 0,9 |
| **Summe** | | 100 | 100 | 100 |

* Vergleichsbeispiel
[a] = Additionsprodukt aus Methacrylsäure und Bisphenol-A-diglycidylether
[b] = Additionsprodukt aus 2-Hydroxyethylmethacrylat und 2,2,4- oder 2,4,4-Trimethylhexamethylen-1,6-diisocyanat
[c] = 2-[4-(2-(Meth)acryloyloxyethoxyethoxy)phenyl]-2-[4-(2-methacryloyloxyethoxy)phenyl]-propan)
[d] = 2,2-Bis[4-(2-methacryloxypropoxy)phenyl]propan
[e] = Bismethacryloyloxymethyltricyclo[5.2.1.]decan
[f] = Bis(4-methoxybenzoyl)diethylgerman
[g] = 4-(Dimethylamino)-benzoesäureester
[h] = gemahlenes Kompositmaterial auf Basis von Bis-GMA[a], UDMA[b], 1,12-Dodecandiioldimethacrylat ($D_3MA$), Benzoylperoxid, silanisiertem Ba-Al-Borosilikatglasfüller (mittlere Partikelgröße 0,4 μm), Ytterbiumfluorid (mittlere Partikelgröße 0,2 μm)
[i] = silanisiertes Bariumaluminiumborosilikatglaspulver (mittlere Partikelgröße von 0,7 μm)
[j] = silanisiertes $SiO_2$-$ZrO_2$-Mischoxid (mittlere Partikelgröße 1,2 μm)
[k] = Ytterbiumfluorid (mittlere Partikelgröße 0,2 μm)
[l] = 2-(Toluol-4-sulfonyloxy)-acrylsäureethylester
[m] = 2-(Toluol-4-sulfonylmethyl)-acrylsäureethylester

***Tabelle 2: Physikalische Eigenschaften der Komposite***

| Eigenschaft | Belichtungszeit [s] | Komposit A* | Komposit B | Komposit C |
|---|---|---|---|---|
| Biegefestigkeit [MPa] | 1 | 105 ± 7 | 115 ± 9 | - |
| | 2 | - | 120 ± 6 | 114 ± 4 |
| | 3 | - | 115 ± 9 | 116 ± 5 |
| | 10 | 100 ± 10 | 119 ± 4 | 119 ± 13 |
| Biegemodul [GPa] | 1 | 9,6 ± 0,5 | 11,3 ± 0,5 | - |
| | 2 | - | 11,2 ± 0,5 | 10,1 ± 0,6 |
| | 3 | - | 11,3 ± 0,5 | 9,8 ± 0,5 |
| | 10 | 9,8 ± 0,5 | 11,2 ± 0,4 | 10,4 ± 0,2 |
| Vickers Härte [MPa] | 1 | 635 / 545 | 650 / 580 | - |
| | 2 | - | 670 / 560 | 660 / 595 |
| | 3 | - | | 623 / 587 |
| | 10 | 640 / 520 | 660 / 570 | 670 / 635 |
| DBU[a] [%] | 1 | 44 ± 3 | 49 ± 2 | - |
| | 2 | - | 53 ± 2 | 49 ± 1 |
| | 3 | - | 47 ± 2 | 45 ± 4 |
| | 10 | 42 ± 2 | 51 ± 2 | 46 ± 1 |

(fortgesetzt)

| Eigenschaft | Belichtungszeit [s] | Komposit A* | Komposit B | Komposit C |
|---|---|---|---|---|
| SK[b] [N] | 1 | 85 ± 5 | 88 ± 9 | - |
| | 2 | - | 86 ± 3 | 78 ± 1 |
| | 3 | - | | 78 ± 3 |
| | 10 | 98 ± 2 | 83 ± 3 | 71 ± 2 |

* Vergleichsbeispiel
[a] Doppelbindungsumsatz
[b] Schrumpfungsspannung

**Beispiel 2:**

*Herstellung von Kompositen mit unterschiedlichem Gehalt an Übertragungsreagenz*

Mit einem Kneter wurden Kompositpasten entsprechend Komposit B aus Beispiel 1 hergestellt, wobei die Menge an eingesetztem Vinylsulfonester **(TSVEA)** variiert wurde (Tabelle 3). Aus den Kompositpasten wurden entsprechende Prüfkörper präpariert, für 3 s (3060 mW/cm$^2$) mit einer Polymerisationslampe (Bluephase G4 (390-510 nm) mit Power Cure (3s) Modus) belichtet und damit ausgehärtet. Anschließend wurden die Biegefestigkeit und das Biege-E-Moduls gemäß ISO-Norm ISO-4049 gemessen (Dentistry - Polymer-based filling, restorative and luting materials) (Tabelle 4). Die Messungen wurden nach 24 h Lagerung in Wasser (37 °C) durchgeführt. Der Doppelbindungsumsatz in 4 mm Tiefe (DBU) wurde mittels FTIR-Spektroskopie bestimmt, die Durchhärtungstiefe (DOC) gemäß ISO 4094 (Tabelle 4).

In Tabelle 4 ist zu erkennen, dass der Komposit E mit 0,7% Übertragungsreagenz insgesamt die besten mechanischen Eigenschaften aufweist.

***Tabelle 3: Zusammensetzung der Komposite***

| Komponente | Komp. D (Gew.-%) | Komp. E (Gew.-%) | Komp. B (Gew.-%) | Komp. F (Gew.-%) |
|---|---|---|---|---|
| Bis-GMA[a] | 9,55 | 9,3 | 9,1 | 8,65 |
| UDMA[b] | 5,0 | 5,0 | 5,0 | 5,0 |
| SR-348c[c] | 2,3 | 2,3 | 2,3 | 2,3 |
| Bis-PMA[d] | 3,5 | 3,5 | 3,5 | 3,5 |
| TCDMA[e] | 1 | 1 | 1 | 1 |
| Ivocerin®[f] | 0,03 | 0,03 | 0,03 | 0,03 |
| Campherchinon | 0,04 | 0,04 | 0,04 | 0,04 |
| EDMAB[g] | 0,13 | 0,13 | 0,13 | 0,13 |
| Isofüller[h] | 18 | 18 | 18 | 18 |
| Glasfüller[i] | 43 | 43 | 43 | 43 |
| $SiO_2$-$ZrO_2$ Sphärosil[j] | 11 | 11 | 11 | 11 |
| $YbF_3$[k] | 6 | 6 | 6 | 6 |
| TSVEA[l] | 0,45 | 0,7 | 0,9 | 1,35 |
| **Summe** | **100** | **100** | **100** | **100** |

Die Bedeutung der Indices [a-l] kann der Tabelle 1 entnommen werden.

***Tabelle 4: Physikalische Eigenschaften der Komposite***

| Eigenschaft | Komposit D | Komposit E | Komposit B | Komposit F |
|---|---|---|---|---|
| Biegefestigkeit [MPa] | 100 ± 13 | 122 ± 5 | 115 ± 9 | 111 ± 6 |

(fortgesetzt)

| Eigenschaft | Komposit D | Komposit E | Komposit B | Komposit F |
|---|---|---|---|---|
| Biegemodul [GPa] | 10,6 ± 0,5 | 11,3 ± 0,4 | 11,3 ± 0,5 | 10,1 ± 0,6 |
| DBU[a] [%] | 50 ± 2 | 50 ± 1 | 47 ± 2 | 54 ± 2 |
| DOC[b] [mm] | 3,0 | 3,1 | 2,9 | 3,0 |

[a] Doppelbindungsumsatz
[b] Durchhärtungstiefe

**Beispiel 3**

*Klinische Randdichtigkeit von Klasse-II-Füllungen*

Die klinische Randdichtigkeit von gelegten Klasse-II-Füllungen wurde in humanen Zähnen unter Verwendung der Komposite A, B und E untersucht. Die Restaurationen wurden in Kombination mit einem dentalen Adhäsiv (Adhese Universal, Fa. Ivoclar Vivadent AG) im self-etch-Modus gelegt. Hierzu wurden in extrahierten Molaren des Unterkiefers je zwei Klasse II-Kavitäten präpariert (koronal-apikale Tiefe 4 mm, bukko-linguale Ausdehnung 5 mm) und mit einer Adhäsiv-/Kompositkombination gefüllt. Am Schmelz beginnend wurde die zu behandelnde Zahnoberfläche vollständig mit Adhäsiv benetzt und das Adhäsiv für mindestens 20 Sekunden auf der zu behandelnden Zahnoberfläche eingerieben. Im Anschluss wurde das Adhäsive mit öl- und wasserfreier Druckluft so lange verblasen, bis ein glänzender, unbeweglicher Film entstanden war. Danach wurde das Adhäsiv gemäß Herstellerangaben durch Bestrahlung mit Licht polymerisiert und dann die Komposite jeweils in einer Schicht in die Kavitäten eingebracht und durch Bestrahlung mit Licht polymerisiert (10 s, 1200 $mW/cm^2$; 3 s, 3060 $mW/cm^2$; 2 s, 5000 $mW/cm^2$; bzw. 1 s, 10000 $mW/cm^2$). Die gefüllten Zähne wurden danach einer künstlichen Alterung mittels Temperaturwechselbadbelastung (10000 x 5°C/55°C, je 30 s) unterzogen. Im Anschluss wurden mittels Abdrücken aus Epoxidharz (Stycast) Replikate angefertigt. Mit dem Rasterelektronenmikroskop wurde bei 200facher Vergrößerung die Randqualität des approximalen Bereichs der Füllung beurteilt, indem der prozentuale Anteil an dichtem Rand in Relation zum Gesamtrand bestimmt wurde.

Aus Tabelle 5 ist zu erkennen, dass die erfindungsgemäßen Komposite B und E deutlich höhere Werte für die Randdichtigkeit ergaben als das Vergleichsmaterial A.

***Tabelle 5: Randdichtigkeit nach TC und KS***

| Eigenschaft | Belichtungs-zeit [s] | Komposit A* | Komposit B | Komposit E |
|---|---|---|---|---|
| % dichter Rand | 1 | 8,9 ± 10,8 | 70,8 ± 8,1 | - |
| | 2 | - | - | 76,3 ± 10,8 |
| | 3 | - | - | 68,9 ± 15,5 |
| | 10 | 18,8 ± 16,3 | - | 64,8 ± 12,1 |

Vergleichsbeispiel

**Patentansprüche**

1. Radikalisch polymerisierbarer Werkstoff, der

(a) 0,01 bis 5 Gew.-% mindestens eines Übertragungsreagenzes,
(b) 5 bis 60 Gew.-% mindestens eines multifunktionellen (Meth)acrylates oder einer Mischung von mono- und multifunktionellen (Meth)acrylaten,
(c) 0,01 bis 3,0 Gew.-% einer Mischung mindestens eines monomolekularen und mindestens eines bimolekularen Photoinitiators,
(d) 30 bis 90 Gew.-% mindestens eines Füllstoffs, und
(e) gegebenenfalls Additiv(e) enthält, jeweils bezogen auf die Gesamtmasse des Werkstoffs,

wobei der Werkstoff als Übertragungsreagenz (a) mindestens ein Allylsulfon der Formel I

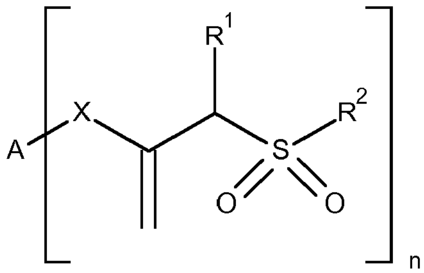

Formel I

,

in der die Variablen die folgenden Bedeutungen haben:

A H; -CN; ein Phenylrest, der einen oder mehrere Substituenten, wie $-CH_3$, $-C_2H_5$, -OH, $-OCH_3$, $-O-COCH_3$, eine polymerisationsfähige Vinyl-, (Meth)acryloyloxy- oder (Meth)acrylamidgruppe tragen kann, oder ein aliphatischer linearer oder verzweigter $C_1$-$C_{20}$-Alkylen- oder $C_1$-$C_{20}$-Alkyl-Rest, der durch ein oder mehrere 1,4-Phenylengruppen, Urethangruppen, O oder S unterbrochen sein kann, der einen oder mehrere Substituenten, wie -OH oder $-OCH_3$, tragen kann und der endständig eine Tri-$C_1$-$C_4$-alkoxysilylgruppe oder eine polymerisationsfähige Vinyl-, (Meth)acryloyloxy- oder (Meth)acrylamidgruppe tragen kann;

$R^1$ jeweils unabhängig voneinander H, ein aliphatischer linearer oder verzweigter $C_1$-$C_9$-Alkyl-Rest, Tolyl oder Phenyl;

$R^2$ jeweils unabhängig voneinander ein Phenylrest, der einen oder mehrere Substituenten, wie $-CH_3$, $-C_2H_5$, -OH, $-OCH_3$, $-O-COCH_3$, eine polymerisationsfähige Vinyl-, (Meth)acryloyloxy-, (Meth)acrylamidgruppe, $-C(=CH_2)-COOR^3$ oder $-C(=CH_2)-CO-NR^4R^5$ tragen kann; oder ein aliphatischer linearer oder verzweigter $C_1$-$C_{20}$-Alkyl-Rest, der durch O oder S unterbrochen sein kann und der endständig eine polymerisationsfähige Vinyl-, (Meth)acryloyloxy-, (Meth)acrylamidgruppe, $-C(=CH_2)-COOR^3$ oder $-C(=CH_2)-CO-NR^4R^5$ tragen kann, wobei $R^{3-5}$ unabhängig voneinander jeweils ein linearer oder verzweigter $C_{1-6}$-Alkyl-Rest sind;

X -COO-, $-CON(R^6)$- oder entfällt, wobei die Bindung an A über O bzw. N erfolgt und wobei

$R^6$ H; oder ein aliphatischer linearer oder verzweigter $C_1$-$C_{20}$-Alkyl-Rest ist, der durch ein oder mehrere O oder S unterbrochen sein kann und der endständig eine polymerisationsfähige Vinyl-, (Meth)acryloyloxy-, (Meth) acrylamidgruppe, $-C(=CH_2)-COOR^3$ oder $-C(=CH_2)-CO-NR^4R^5$ tragen kann, wobei $R^{3-5}$ unabhängig voneinander jeweils ein linearer oder verzweigter $C_{1-6}$-Alkyl-Rest sind;

n eine ganze Zahl von 1 bis 6,

und/oder
einen Vinylsulfonester der Formel II enthält,

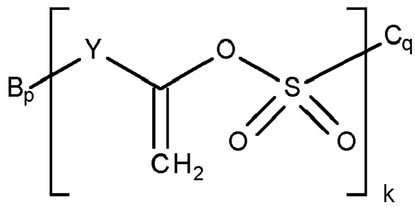

Formel II

,

in der die Variablen die folgenden Bedeutungen haben:

B H, CN, ein cycloaliphatischer, linearer oder verzweigter aliphatischer $C_1$-$C_{30}$-Kohlenwasserstoffrest, der durch einen oder mehrere, vorzugsweise 1 bis 6, besonders bevorzugt 1 bis 3 Substituenten, vorzugsweise $-CH_3$, $-C_2H_5$, -OH, $-OCH_3$ und/oder $-O-COCH_3$, substituiert sein kann, der durch eine oder mehrere, vorzugsweise 1 bis 4, besonders bevorzugt 1 bis 2 Urethangruppen, Estergruppen, O und/oder S unterbrochen sein kann und der 1 bis 4, vorzugsweise 1 oder 2 Benzolgruppen enthalten kann, ein aromatischer $C_6$-$C_{30}$-Kohlenwasserstoffrest, der durch einen oder mehrere, vorzugsweise 1 bis 6, besonders bevorzugt 1 bis 3 Substituenten, vorzugsweise $-CH_3$, $-C_2H_5$, -OH, $-OCH_3$ und/oder $-O-COCH_3$, substituiert sein kann, oder eine Kombination davon;

Y -COO-, $-CON(R^7)$- oder entfällt, wobei die Bindung an B über O oder N erfolgt;

C ein cycloaliphatischer, linearer oder verzweigter aliphatischer $C_1$-$C_{30}$-Kohlenwasserstoffrest, der durch einen

oder mehrere, vorzugsweise 1 bis 6, besonders bevorzugt 1 bis 3 Substituenten, vorzugsweise $C_1$-$C_5$-Alkyl, -OH, -O-$COCH_3$ und/oder $C_1$-$C_5$-Alkoxy, substituiert sein kann, der durch eine oder mehrere, vorzugsweise 1 bis 4, besonders bevorzugt 1 bis 2 Urethangruppen, Estergruppen, O und/oder S unterbrochen sein kann und der 1 bis 4, vorzugsweise 1 oder 2 Benzolgruppen enthalten kann, ein aromatischer $C_6$-$C_{30}$-Kohlenwasserstoffrest, der durch einen oder mehrere, vorzugsweise 1 bis 6, besonders bevorzugt 1 bis 3 Substituenten, vorzugsweise Vinyl, $C_1$-$C_5$-Alkyl, -OH, -O-$COCH_3$ und/oder $C_1$-$C_5$-Alkoxy, substituiert sein kann, oder Tosyl, oder eine Kombination davon;

$R^7$ Wasserstoff, ein cycloaliphatischer, linearer oder verzweigter aliphatischer $C_1$-$C_{10}$-Kohlenwasserstoffrest, der durch eine oder mehrere, vorzugsweise 1 bis 4, besonders bevorzugt 1 bis 2 Sauerstoffatome unterbrochen sein kann und der durch eine oder mehrere, vorzugsweise 1 bis 4, besonders bevorzugt 1 bis 2 OH-Gruppen substituiert sein kann, oder ein aromatischer $C_6$-$C_{10}$-Kohlenwasserstoffrest, der durch eine oder mehrere, vorzugsweise 1 bis 4, besonders bevorzugt 1 bis 2 OH-Gruppen substituiert sein kann;

p eine ganze Zahl von 1 bis 6;

k eine ganze Zahl von 1 bis 6;

q eine ganze Zahl von 1 bis 6; wobei

p und q nicht gleichzeitig größer als 1 sein können und wobei dann, wenn p = 1 ist, q = k ist, und wenn q = 1 ist, p = k ist.

**2.** Werkstoff nach Anspruch 1, in dem die Variablen der Formel I die folgenden Bedeutungen haben:

A H; ein aliphatischer linearer oder verzweigter $C_1$-$C_{14}$-Alkylen- oder $C_1$-$C_{14}$-Alkyl-Rest, der durch ein oder mehrere O oder S unterbrochen sein kann, der einen oder mehrere Substituenten, wie -OH oder -$OCH_3$, tragen kann und der endständig eine Tri-$C_1$-$C_3$-alkoxysilylgruppe oder eine polymerisationsfähige Vinyl-, (Meth)acryloyloxy- oder (Meth)acrylamidgruppe tragen kann;

$R^1$ H, ein aliphatischer verzweigter oder vorzugsweise linearer $C_1$-$C_4$-Alkyl-Rest, Tolyl oder Phenyl;

$R^2$ jeweils unabhängig voneinander ein Phenylrest, der einen oder mehrere Substituenten, wie -$CH_3$, -$C_2H_5$, -OH, -$OCH_3$, -O-$COCH_3$, tragen kann; oder ein aliphatischer linearer oder verzweigter $C_1$-$C_{10}$-Alkyl-Rest, der durch O oder S unterbrochen sein kann;

X -COO-, -CON($R^6$)- oder entfällt, wobei die Bindung an A über O oder N erfolgt und wobei

$R^6$ H; oder ein aliphatischer linearer oder verzweigter $C_1$-$C_{10}$-Alkyl-Rest, der durch ein oder mehrere O oder S unterbrochen sein kann;

n eine ganze Zahl von 1 bis 3,

und/oder
in dem die Variablen der Formel II die folgenden Bedeutungen haben:

B H, ein cycloaliphatischer, linearer oder verzweigter aliphatischer $C_1$-$C_{15}$-Kohlenwasserstoffrest, der durch 1 bis 4, besonders bevorzugt 1 bis 3 Substituenten, vorzugsweise -$CH_3$, -$C_2H_5$, -OH, -$OCH_3$ und/oder -O-$COCH_3$, substituiert sein kann, der durch 1 bis 4, besonders bevorzugt 1 bis 2 Urethangruppen, Estergruppen, O und/oder S unterbrochen sein kann und der 1 oder 2 Benzolgruppen enthalten kann, ein aromatischer $C_6$-$C_{18}$-Kohlenwasserstoffrest, der durch 1 bis 6, besonders bevorzugt 1 bis 3 Substituenten, vorzugsweise -$CH_3$, -$C_2H_5$, -OH, -$OCH_3$ und/oder -O-$COCH_3$, substituiert sein kann, oder eine Kombination davon;

Y -COO-, -CON($R^7$)- oder entfällt, wobei die Bindung an B über O oder N erfolgt;

C ein cycloaliphatischer, linearer oder verzweigter aliphatischer $C_1$-$C_{15}$-Kohlenwasserstoffrest, der durch 1 bis 4, besonders bevorzugt 1 bis 3 Substituenten, vorzugsweise $C_1$-$C_4$-Alkyl, -OH, -O-$COCH_3$ und/oder $C_1$-$C_4$-Alkoxy, substituiert sein kann, der durch 1 bis 4, besonders bevorzugt 1 bis 2 Urethangruppen, Estergruppen, O und/oder S unterbrochen sein kann und der 1 oder 2 Benzolgruppen enthalten kann, ein aromatischer $C_6$-$C_{18}$-Kohlenwasserstoffrest, der durch 1 bis 6, besonders bevorzugt 1 bis 3 Substituenten, vorzugsweise Vinyl, $C_1$-$C_4$-Alkyl, -OH, -O-$COCH_3$ und/oder $C_1$-$C_4$-Alkoxy, substituiert sein kann, oder Tosyl, oder eine Kombination davon;

$R^7$ Wasserstoff, ein cycloaliphatischer, linearer oder verzweigter aliphatischer $C_1$-$C_6$-Kohlenwasserstoffrest, der durch eine oder mehrere, vorzugsweise 1 bis 2 Sauerstoffatome unterbrochen sein kann und der durch eine oder mehrere, vorzugsweise 1 bis 2 OH-Gruppen substituiert sein kann, oder ein aromatischer $C_6$-$C_{10}$-Kohlenwasserstoffrest, der durch eine oder mehrere, vorzugsweise 1 bis 2 OH-Gruppen substituiert sein kann;

p eine ganze Zahl von 1 bis 3;

k eine ganze Zahl von 1 bis 3;

q eine ganze Zahl von 1 bis 3; wobei

p und q nicht gleichzeitig größer als 1 sein können und wobei dann, wenn p = 1 ist, q = k ist, und wenn q = 1 ist, p = k ist.

3. Werkstoff nach Anspruch 2, der als Übertragungsreagenz gemäß der Formel I 2-(Toluol-4-sulfonylmethyl)-acrylsäure-ethylester (TSMEA), 2-(Toluol-4-sulfonylmethyl)-acrylsäure-2-(2-ethoxyethoxy)-ethylester, 2-(Toluol-4-sulfonylmethyl)-acrylsäure, 2-(Toluol-4-sulfonylmethyl)-acrylsäuretert-butylester, 3-(Trimethoxysilyl)propyl-2-(tosylmethyl)acrylat, 3-(Triethoxysilyl)propyl-2-(tosylmethyl)acrylat, 3-(Triethoxysilyl)propyl-2-(tosylmethyl)acrylamid, 2-(Methylsulfonylmethyl)-ethylacrylat, 2-(Methylsulfonylmethyl)-propylacrylat, Triethylenglycol-bis[2-(toluol-4-sulfonylmethyl)-acrylat], 2-(Toluol-4-sulfonylmethyl)-acrylsäure-(2-methacryloyloxyethyl)ester, 2-(Toluol-4-sulfonylmethyl)-acrylsäure-(6-methacryloyloxyhexyl)ester, 2-(Toluol-4-sulfonylmethyl)-acrylsäure-(10-methacryloyloxydecyl)ester, 2-(Toluol-4-sulfonylmethyl)-acrylsäure-(2-hydroxy-3-methacryloyloxypropyl)ester, 2-(Toluol-4-sulfonylmethyl)-acrylsäure-(8-methacryloyloxy-3,6-dioxaoctyl)ester, vorzugsweise 2-(Toluol-4-sulfonylmethyl)-acrylsäureethylester (TSMEA),
und/oder
als Übertragungsreagenz gemäß der Formel II 2-(Toluol-4-sulfonyloxy)-acrylsäureethylester (TSVEA), 2-Methansulfonyloxyacrylsäureethylester, Triethylenglykol-bis[2-(toluol-4-sulfonyloxy)-acrylat], 2-(2,4,6-Triisopropylbenzolsulfonyloxy-acrylsäureoctylester, 2-(Toluol-4-sulfonyloxy)-acrylsäure-2-(tetrahydropyran-2-yloxy)-ethylester, 2-(Toluol-4-sulfonyloxy)-acrylsäure-2-hydroxyethylester, 2-(Toluol-4-sulfonyloxy)-acrylsäure-2,4,6-trimethylphenylester, 2-(4-Vinylbenzolsulfonyloxy)-acrylsäureethylester, 2-(Toluol-4-sulfonyloxy)-acrylsäuremethoxymethylester, 2-(2,4,6-Triisopropylbenzolsulfonyloxy)-acrylsäuremethoxymethylester, 2-(2,4,6-Triisopropylbenzolsulfonyloxy)-acrylsäure oder Diethyl-2,2'-[(1,3-phenyldisulfonyl)bis(oxy)]-diacrylat enthält, vorzugsweise 2-(Toluol-4-sulfonyloxy)-acrylsäureethylester (TSVEA) enthält.

4. Werkstoff nach einem der vorhergehenden Ansprüche, der als Komponente (b) mindestens ein multifunktionelles (Meth)-acrylat ausgewählt aus der Gruppe bestehend aus Bisphenol-A-dimethacrylat, Bis-GMA (ein Additionsprodukt aus Methacrylsäure und Bisphenol-A-diglycidylether), 2-[4-(2-(Meth)acryloyloxyethoxyethoxy)phenyl]-2-[4-(2-methacryloyloxyethoxy)phenyl]-propan), 2,2-Bis[4-(2-methacryloxypropoxy)phenyl]propan (Bis-PMA), UDMA (ein Additionsprodukt aus 2-Hydroxyethylmethacrylat und 2,2,4- oder 2,4,4-Trimethylhexamethylen-1,6-diisocyanat) und Bismethacryloyloxymethyltricyclo[5.2.1.]decan (TCDMA) enthält.

5. Werkstoff nach einem der vorhergehenden Ansprüche, der als monomolekularen Photoinitiator eine Diacyldialkylgermanium- oder Tetraacylgermanium-Verbindung, ein Mono- oder Bisacylphosphinoxid oder eine Mischung davon, und als bimolekularen Photoinitiator Campherchinon (CQ), 9,10-Phenanthrenchinon, 1-Phenyl-propan-1,2-dion, 2,2-Dimethoxy-2-phenyl-acetophenon, Diacetyl, 4,4'-Dichlorbenzil oder eine Mischung davon, vorzugsweise in Kombination mit einem Amin, vorzugsweise in Kombination mit 4-(Dimethylamino)-benzoesäureester (EDMAB), N,N-Dimethylaminoethylmethacrylat, N,N-Dimethyl-sym.-xylidin, Triethanolamin oder einer Mischung davon enthält.

6. Werkstoff nach einem der vorhergehenden Ansprüche, der als monomolekularen Photoinitiator 2,4,6-Trimethylbenzoyldiphenylphosphinoxid, Bis(2,4,6-trimethylbenzoyl)phenylphosphinoxid, Dibenzoyldiethylgerman, Bis(4-methoxybenzoyl)-diethylgerman (MBDEGe), Tetrabenzoylgerman, Tetrakis(o-methylbenzoyl)german, Tetrakis(mesitoyl)stannan oder eine Mischung davon, als bimolekularen Photoinitiator Campherchinon (CQ), 2,2-Dimethoxy-2-phenyl-acetophenon oder eine Mischungen davon und als Amin 4-(Dimethylamino)-benzoesäureester (EDMAB) enthält.

7. Werkstoff nach einem der vorhergehenden Ansprüche, bei dem der mindestens eine monomolekulare Photoinitiator und der mindestens eine bimolekulare Photoinitiator in einem Gewichtsverhältnis von 2:1 bis 1:2, vorzugsweise von 1,4:1 bis 1:2, besonders bevorzugt von 1,2:1 bis 1:1,8, und ganz besonders bevorzugt von 1:1 bis 1:1,8 eingesetzt werden.

8. Werkstoff nach einem der vorhergehenden Ansprüche, bei dem der bimolekulare Photoinitiator und das Amin in einem Gewichtsverhältnis von 1:1 bis 1:6 eingesetzt werden.

9. Werkstoff nach einem der vorhergehenden Ansprüche, der als Füllstoff (d) ein Mischoxid aus $SiO_2$ und $ZrO_2$, ein Glaspulver, vorzugsweise Barium- oder Strontiumaluminiumsilikatglaspulver, einen röntgenopaken Füllstoff, vorzugsweise Ytterbiumtrifluorid oder ein Mischoxid von $SiO_2$ mit Ytterbium(III)-oxid, ein gemahlenes Präpolymerisat oder eine Mischung davon enthält.

10. Werkstoff nach einem der vorhergehenden Ansprüche, der

(a) 0,1 bis 5 Gew.-%, bevorzugt 0,1 bis 3 Gew.-% mindestens eines Übertragungsreagenzes der Formel(n) I und/oder II,
(b) 8 bis 50 Gew.-%, bevorzugt 10 bis 40 Gew.-% mindestens eines multifunktionellen (Meth)acrylates oder einer Mischung von mono- und multifunktionellen (Meth)-acrylaten,
(c) 0,05 bis 3,0 Gew.-% und bevorzugt 0,1 bis 2,0 Gew.-% einer Mischung mindestens eines monomolekularen und mindestens eines bimolekularen Photoinitiators,
(d) 40 bis 90 Gew.-% und bevorzugt 50 bis 85 Gew.-% mindestens eines Füllstoffs, und
(e) 0 bis 5 Gew.-%, bevorzugt 0 bis 3 Gew.-% und besonders bevorzugt 0,2 bis 3 Gew.-% Additiv(e) enthält,

jeweils bezogen auf die Gesamtmasse des Werkstoffs.

**11.** Werkstoff nach Anspruch 10, der

(a) 0,1 bis 5 Gew.-%, bevorzugt 0,1 bis 3 Gew.-% mindestens eines Übertragungsreagenzes der Formel(n) I und/oder II,
(b) 10 bis 40 Gew.-% mindestens eines multifunktionellen (Meth)acrylates ausgewählt aus Bisphenol-A-dimethacrylat, Bis-GMA (ein Additionsprodukt aus Methacrylsäure und Bisphenol-A-diglycidylether), 2-[4-(2-(Meth)acryloyloxyethoxyethoxy)phenyl]-2-[4-(2-methacryloyloxyethoxy)phenyl]-propan), 2,2-Bis[4-(2-methacryloxypropoxy)phenyl]propan (Bis-PMA), UDMA (ein Additionsprodukt aus 2-Hydroxyethylmethacrylat und 2,2,4- oder 2,4,4-Trimethylhexamethylen-1,6-diisocyanat), Bis-methacryloyloxymethyltricyclo[5.2.1.]decan (TCDMA) und Mischungen davon,
(c) 0,1 bis 2,0 Gew.-% einer Mischung einer Diacyldialkylgermanium-, Tetraacylgermanium- und/oder Tetraacylstannan-Verbindung oder eines Mono- oder Bisacylphosphinoxids mit einem bimolekularen Photoinitiator, vorzugsweise Campherchinon (CQ) und/oder 2,2-Dimethoxy-2-phenyl-acetophenon, sowie mit 4-(Dimethylamino)-benzoesäureester (EDMAB),
(d) 50 bis 85 Gew.-% Füllstoff ausgewählt aus Mischoxiden von $SiO_2$ und $ZrO_2$, Glaspulvern, vorzugsweise Barium- oder Strontiumaluminiumsilikatglaspulvern, röntgenopaken Füllstoffen, vorzugsweise Ytterbiumtrifluorid und Mischoxiden von $SiO_2$ mit Ytterbium(III)-oxid, gemahlenen Präpolymerisaten und Mischungen davon, und
(e) 0,2 bis 3 Gew.-% Additiv(e) enthält,

jeweils bezogen auf die Gesamtmasse des Werkstoffs.

**12.** Werkstoff nach einem der Ansprüche 1 bis 11 zur intraoralen Verwendung zur Restauration geschädigter Zähne, vorzugsweise zur intraoralen Verwendung als dentaler Zement, Füllungskomposit oder Verblendmaterial.

**13.** Verwendung eines Werkstoffs nach einem der Ansprüche 1 bis 11 als Material zur extraoralen Herstellung oder Reparatur von Dentalrestaurationen.

**14.** Verwendung nach Anspruch 13 zur Herstellung von künstlichen Zähnen, Prothesen, Inlays, Onlays, Kronen oder Brücken.

**15.** Verwendung eines Werkstoffs nach einem der Ansprüche 1 bis 11 zur Herstellung von Formkörpern durch Gießen, Pressen oder 3D-Druck.

Europäisches Patentamt
European Patent Office
Office européen des brevets

# EUROPÄISCHER RECHERCHENBERICHT

Nummer der Anmeldung
EP 18 20 8916

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | EP 2 965 741 A1 (IVOCLAR VIVADENT AG [LI]; TECH UNIVERSITÄT WIEN [AT]) 13. Januar 2016 (2016-01-13) * Ansprüche 1-16 * * Seite 3, Absatz 12 * * Seite 7, Absatz 27 - Absatz 30 * * Seite 8, Absatz 34 * ----- | 1-15 | INV. A61K6/083 C08F2/38 |
| X,D | EP 3 091 037 A1 (IVOCLAR VIVADENT AG [LI]; TECH UNIVERSITÄT WIEN [AT]) 9. November 2016 (2016-11-09) * Ansprüche 1-17 * * Seite 10, Absatz 42 - Absatz 45 * * Seite 10, Absatz 49 * ----- | 1-15 | |
| X | K. SEIDLER ET AL: "Vinyl Sulfonate Esters: Efficient Chain Transfer Agents for the 3D Printing of Tough Photopolymers without Retardation", ANGEWANDTE CHEMIE , INTERNATIONAL EDITION, Bd. 57, 4. Mai 2018 (2018-05-04), Seiten 9165-9169, XP002791070, DOI: 10.1002/anie.201803747 * Zusammenfassung * * Seite 9166, rechte Spalte * * Seite 9169, linke Spalte, letzter Absatz * ----- -/-- | 1-15 | RECHERCHIERTE SACHGEBIETE (IPC) A61K C08F |

Der vorliegende Recherchenbericht wurde für alle Patentansprüche erstellt

1

| Recherchenort | Abschlußdatum der Recherche | Prüfer |
|---|---|---|
| Berlin | 7. Mai 2019 | Siatou, Evangelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 18 20 8916

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | C. GORSCHE ET AL: "Exploring the benefits of beta-allyl sulfones for more homogeneous dimethacrylate photopolymer networks", POLYMER CHEMISTRY, Bd. 6, 2015, Seiten 2038-2047, XP002791071, * Zusammenfassung * * Seite 2041; Abbildung 2 * * Seite 2046, rechte Spalte, letzter Absatz * ----- | 1-15 | |
| X | C. GORSCHE ET AL: "Rapid formation of regulated methacrylate networks yielding tough materials for lithography-based 3D printing", POLYMER CHEMISTRY, Bd. 7, 2009, Seiten 2009-2014, XP002791072, * Zusammenfassung * ----- | 1-15 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Berlin | 7. Mai 2019 | Siatou, Evangelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 18 20 8916

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-05-2019

| Im Recherchenbericht angeführtes Patentdokument | Datum der Veröffentlichung | Mitglied(er) der Patentfamilie | Datum der Veröffentlichung |
|---|---|---|---|
| EP 2965741 A1 | 13-01-2016 | CN 106535862 A | 22-03-2017 |
| | | EP 2965741 A1 | 13-01-2016 |
| | | EP 3166569 A1 | 17-05-2017 |
| | | JP 2017523161 A | 17-08-2017 |
| | | US 2017172855 A1 | 22-06-2017 |
| | | WO 2016005534 A1 | 14-01-2016 |
| EP 3091037 A1 | 09-11-2016 | EP 3091037 A1 | 09-11-2016 |
| | | EP 3292157 A1 | 14-03-2018 |
| | | US 2018142082 A1 | 24-05-2018 |
| | | WO 2016177677 A1 | 10-11-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20120295228 A1
- WO 2015057413 A1
- WO 2015041863 A1
- EP 2965741 A1
- EP 3090772 A1

### In der Beschreibung aufgeführte Nicht-Patentliteratur

- **R. R. BRAGA ; R. Y. BALLESTER ; J. L. FERRACANE.** *Dent. Mater.,* 2005, vol. 21, 962-970
- **J. W. STANSBURY.** *Dent. Mater.,* 2012, vol. 28, 13-22